# EUROPEAN PATENT SPECIFICATION

(11) **EP 3 547 968 B1**
(45) Date of publication and mention of the grant of the patent: **19.03.2025**
(21) Application number: 17877212.5
(22) Date of filing: 04.12.2017
(51) Int. Cl.: A61F 2/24

(54) **ENGINEERED TISSUE PROSTHESIS**
GEZÜCHTETE GEWEBEPROTHESE
PROTHÈSE OBTENUE PAR GÉNIE TISSULAIRE

(30) Priority: 02.12.2016 US 201662429716 P; 06.03.2017 US 201762467801 P; 17.05.2017 US 201762507758 P
(43) Date of publication of application: 09.10.2019
(73) Proprietor: Vascudyne, LLC, Plano, Texas 75093 (US); Murphy, Richard F., White Bear Townshp, Minnesota 55110 (US); Schankereli, Kemal, Stillwater, Minnesota 55082 (US); Davis, Michael L., Shorewood, Minnesota 55331 (US)
(72) Inventor: MURPHY, Richard F., White Bear Townshp Minnesota 55110 (US); SCHANKERELI, Kemal, Stillwater Minnesota 55082 (US); DAVIS, Michael L., Shorewood Minnesota 55331 (US)
(74) Representative: Tomkins & Co
(86) International application number: PCT/US2017/064559
(87) International publication number: WO 2018/102826

(56) References cited:
- EP-A1- 1 278 559
- US-A1- 2003 228 692
- US-A1- 2003 229 394
- US-A1- 2004 206 363
- US-A1- 2006 210 597
- US-A1- 2009 062 907
- US-A1- 2009 254 175
- US-A1- 2015 088 247
- US-A1- 2015 088 247
- US-A1- 2015 305 860
- US-A1- 2016 158 007
- US-B1- 6 666 886

## Description

### I. Field of the Invention

The invention relates to an engineered tissue venous valve as defined in claim 1. The present disclosure relates to vascular medical devices, systems and methods; and more particularly to venous valves including a venous valve frame comprising engineered tissue, and methods for forming and using the venous valve frame.

### II. Background of the Invention

The venous system uses valves and muscles as part of the body's pumping mechanism to return blood to the heart. Venous valves create one way flow to prevent blood from flowing away from the heart. When a valve fails in the heart, blood pumped to the lungs may be less efficient, and blood flow in the atrium and vena cavaes may be affected. When valves fail in the legs, blood can pool in the lower legs resulting in swelling and ulcers of the leg.

The absence of functioning venous valves can lead to chronic venous insufficiency, venous hypertension, chronic venous insufficiency, deep vein thrombosis, or a host of other conditions or diseases.

Techniques for both repairing and replacing the valves exist, but are tedious and require invasive surgical procedures. Direct and indirect valvuoplasty procedures are used to repair damaged valves. Transposition and transplantation are used to replace an incompetent valve. Transposition involves moving a vein with an incompetent valve to a site with a competent valve. Transplantation replaces an incompetent valve with a harvested valve from another venous site.

Prosthetic valves can be transplanted into the venous system, but current devices are not successful enough to see widespread usage. One reason for this is the very high percentage of prosthetic valves reported with leaflet functional failures. These failures have been blamed primarily on improper sizing and tilted deployment of the prosthetic valve. In addition, a great number of leaflets of the prosthetic valves ultimately become fused to the vein wall.

The use of engineered tissue and vascular medical devices configured for a specific venous location address many if not all of the deficiencies in commercially available devices.

The prosthesis of the present invention further includes one or more optional elements that alleviate potential thrombosis in the valve and prevents thickening in specific areas of the leaflets.

Valves within the venous system are configured in a variety of shapes that depend on anatomical location, vessel size, and function. For example, the typical shape of the venous valve in man includes two flaps, a.k.a. cusps or leaflets having free edges that sealingly meet, when closed, to form a commissure. Venous valves are typically associated with a broadened area of the vein forming a sinus pocket behind each leaflet.

Chronic venous insufficiency (CVI) occurs in patients that have deep and superficial venous valves of their lower extremities (distal to their pelvis) that have failed or become incompetent due to congenital valvular abnormalities and/or pathophysiologic disease of the vasculature. As a result, such patients suffer from varicose veins, swelling and pain of the lower extremities, edema, hyper pigmentation, lipodermatosclerosis, and deep vein thrombosis (DVT). Such patients are at increased risk for development of soft tissue necrosis, ulcerations, pulmonary embolism, stroke, heart attack, and amputations.

After a venous valve segment becomes incompetent, the vessel wall dilates and fluid velocity decreases, which may lead to flow stasis and thrombus formation in the proximity of the venous valve. Repair and replacement of venous valves presents a formidable challenge due to the low blood flow rate found in native veins, the very thin wall structure of the venous wall and the venous valve, and the ease and frequency of which venous blood flow can be impeded or totally blocked for a period of time. US 2006/210597 A1 discloses a venous valve which is made from naturally-derived ECM materials.

Surgical reconstruction techniques used to address venous valve incompetence include venous valve bypass using a segment of vein with a competent valve, venous transposition to bypass venous blood flow through a neighboring competent valve, and valvuloplasty to repair the valve cusps. These surgical approaches may involve placement of synthetic, allograft and/or xenograft prostheses inside of or around the vein. However, such prostheses have not been devoid of problems, such as thrombus formation and valve failure due to the implanted prostheses causing non-physiologic flow conditions and/or excessive dilation of the vessels with a subsequent decrease in blood flow rates.

Percutaneous transluminal methods for treatment of venous insufficiency are being studied, some of which include placement of synthetic, allograft and/or xenograft prosthesis that suffer from similar problems as the surgically implanted ones discussed above. In light of these limitations, there is a still a need in the art for an improved device that may be percutaneously placed within a vein having an existing insufficient venous valve to re-establish proper flow through the vein segment.

### III. Summary of the Invention

An apparatus of the present invention is a medical device comprising a valve supported by or attached to a stent, said stent being configured and/or shaped to the anatomical structure in the venous system, wherein said medical device comprises engineered tissue. In preferred embodiments of the invention, the stent and valve are configured for placement in the common femoral vein. A prosthesis of the present invention prevents or reduces the effect of any venous hypertension, regurgitation, backflow, or failure of the native valve. Some embodiments of the invention may be used when there is no native valve failure or insufficiency.

Embodiments of the invention may be used to treat any organ lumen or body lumen having a tubular or tube-like structure.

In some embodiments of the invention, the medical device is a stent or framework supporting or comprising engineered tissue. Some embodiments of the invention may be used in or to treat any condition in a body lumen or vascular system, including but not limited to opening or supporting a body lumen or treating an aneurysm.

Some embodiments of the disclosure are also directed to methods of controlling a body fluid in a tubular or tube-like structure of the body (e.g., containing a lumen), including but not limited to a method of controlling blood flow through a vein, or controlling fluid through the ureter or urethra. A prosthetic venous valve may be percutaneously delivered to a treatment site within the vein or lumen. The prosthetic valve includes a self-expanding tubular body defining a fluid passageway and a pair of opposing leaflets coupled within the fluid passageway of the tubular body. The prosthetic venous valve is deployed at the treatment site. The free edges of the valve leaflets are configured to diverge in response to a pressure differential to form an outflow opening having a substantially elliptical shape that allows blood flow through the fluid passageway of the tubular body.

The disclosure also includes a method for treating native venous valve failure or incompetency, comprising implanting stent that comprises engineered tissue.

The disclosure also includes a method of treating tri-cuspid valve failure or incompetency by positioning a stent comprising engineered tissue in a portion of the inferior vena cava, the superior vena cava, or combinations thereof; said stent be configured to the anatomy in each location. In some embodiments of the disclosure, the prosthesis or stent may be isodiametric or paradiametric, e.g., having two or more different diameters.

In preferred embodiments of the disclosure, the stent, when expanded, is shape formed and adapted to conform to the anatomy when properly positioned. In preferred embodiments of the invention, a prosthesis or stent may include zones or sections having different attributes or characteristics (e.g., flexibility), explained in more detail below.

Devices and methods of the present disclosure may also include structures or elements for retrieving or repositioning the medical device.

An attribute of a stent of the present disclosure includes but is not limited to its flexibility and accommodating a bend while retaining its internal diameter, shape, and configuration. Attributes include, but are not limited to compression resistance, chronic outward radial force, and hoop strength.

With the following enabling description of the drawings, the apparatus should become evident to a person of ordinary skill in the art.

### IV. Brief Description of the Figures

Figure 1 is an exemplary embodiment of a medical device of the present invention, showing a wireform positioned in a stent, and leaflets attached to the wireform.
Figure 2 is an exemplary stent of a medical device of the present invention.
Figure 3 is an exemplary leaflet wireform of a medical device of the present invention. Figure 3A is the bare wireform; Figure 3B shows leaflets attached to the wireform.
Figure 4 is an exemplary embodiment of a medical device of the present invention, showing a cross section of the prosthesis of Figure 3, and an embodiment including a skirt.
Figure 5 is an exemplary embodiment of a medical device of the present invention, showing a cross section of the prosthesis of Figure 3, and an embodiment without a skirt.
Figure 6 is an exemplary embodiment of a medical device of the present invention in which the stent and valve configuration includes a sinus. Fig. 6 A is a top view and Fig. 6B is a side view.
Figure 7 is an exemplary embodiment of a medical device of the present invention showing a stent configured to produce a sinus.
Figure 8 is an exemplary embodiment of a medical device intended for placement in the vena cava.
Figure 9 is an end view of the embodiment shown in Figures 8 and 9.
Figure 10 is an illustration of the right atrium and shows placement of an embodiment of the invention in the superior vena cava and/or the inferior vena cava.

### V. Detailed Description of the Invention

The present disclosure is a prosthesis comprising a stent or framework, and a skirt disposed on the inside lumen of the stent, wherein the skirt is formed from engineered tissue. Some embodiments of the invention may optionally further include one or more of the following: an anchor; a valve with a supporting wireform; a valve without a wireform; and one or more elements configured to retrieve or reposition the stent/prosthesis.

The present disclosure may also be a prosthesis comprising a stent or latticework supporting at least one leaflet or valve, wherein said leaflet or valve is made from engineered tissue. In preferred embodiments, the leaflets may be cut or stamped from engineered tissue (e.g., from a sheet of engineered tissue); or the leaflets may be formed by seeding engineered tissue into a framework or the like.

Embodiments of the present disclosure are directed to vascular medical devices, systems and methods for valve replacement and/or augmentation. Particularly, the present disclosure provides venous valve frames, venous valves that utilize the venous valve frames, and methods for forming and using the venous valve frame and the venous valve. Various embodiments of the present disclosure can be used to replace and/or augment an incompetent valve in a body lumen.

Embodiments of the venous valve include a venous valve frame and valve leaflets that can be implanted through minimally-invasive techniques into a body lumen. The medical devices of the present invention include one or more elements made from engineered tissue, including but not limited to valve leaflets, sewing cuffs, and stent covers.

In one example, embodiments of the apparatus, system, and method for valve replacement or augmentation may help to maintain antegrade blood flow, while decreasing retrograde blood flow in a venous system of individuals having venous insufficiency, such as venous insufficiency in the legs.

Use of valve embodiments can also be possible in other portions of the vasculature.

Use of valve embodiments and non-valve embodiments may also be used in other tubular structures or lumens of the anatomy, including but not limited to ureter and urethra.

The medical devices, engineered tissue, stents, and frameworks of the present invention may be shaped and/or treated to mimic the shape and/or function of a native valve, preferably a native venous valve.

The present invention is a prosthesis for implantation in the venous system, said prosthesis comprising engineered tissue. In some embodiments of the invention, the prosthesis comprises engineered tissue covering a portion or all of the prosthesis framework or stent. Exemplary embodiments may include, but are not limited to a sewing cuff; a stent cover; a skirt covering a portion of an the inside wall of the stent; a skirt seeded or layered on one or more struts of the stent; and a skirt seeded or layered on a section of the stent.

In some embodiments of the invention, the prosthesis includes engineered tissue formed into a valve. Some embodiments may further include a sewing cuff or ring, or the like, any or all of which may be formed from engineered tissue.

In accordance with the present invention, any prosthesis or parts thereof may be formed using engineered tissue. Engineered tissue, sometimes referred to as regenerative tissue, as used herein, refers to tissue formed from cells producing an extracellular matrix. Typically, prior to further processing, the tissue is decellularized. The resulting engineered tissue may be characterized by its lack of crosslinking and its lack of generating an immune response. According to the invention, the engineered tissue includes collagen or fibrous structures that are aligned.

Broadly, some embodiments include combining matrix-producing cells, fibrinogen, and thrombin to produce a cell-seeded composition. The cell-seeded composition is then cultured in an appropriate cell culture medium, and then the cultured cells are decellularized.

It is well known to form or shape the cell-seeded composition on a scaffold or mandrel; such processes result in a tube, shaped leaflets, or an intact valve. In accordance with the present invention, the cell-seeded composition may be formed into a sheet or other configuration.

The engineered tissue of the present invention is distinct from certain other kinds of engineered tissue that does not involve an extra cellular matrix in its production and/or includes cells in its final product.

Without intending to be limited to a particular process for producing engineered tissue, an exemplary method includes combining fibrinogen or the like, thrombin or the like, and extracellular matrix (ECM)-producing cells; and allowing the cells to grow sufficiently to produce ECM. One skilled in the art will recognize that up to nine passages typically optimizes ECM production. According to the invention, the result is cells in a suspension or gel.

The suspension/gel may then be placed in contact with a form, mandrel, substrate, or the like that allows the tissue to be shaped or allows a substrate to be seeded. For example, a tube-shaped mandrel may be formed from a lubricious substance, or may be coated with a lubricious substance (e.g., teflon or pluronic acid); and the suspension/gel may be coated on the mandrel. In some embodiments of the disclosure, two opposing ends of the mandrel may include a slidable anchor (e.g., mesh) or the like that controls the time and amount of contraction of the gel. It has been found that as the tissue/suspension/gel contracts in a controlled fashion, fibers may orient in a certain direction. While on the form or substrate, the gel containing cells may be nourished by adding nutrient, growth, and/or maintenance medium, any or all of which may be chosen to promote ECM production.

After a sufficient amount of time and/or tissue development, the mandrel, form, or substrate containing cells may then be decellularized. A sufficient amount of time, as that phrase is used herein, refers to finding or developing one or more desirable characteristics, including but not limited to opaqueness, stiffness, thickness, tensile strength, and/or fiber alignment.

Exemplary processes for decellularization include but are not limited to washing the tissue in one or more detergents (e.g., Triton and/or SDS), followed by one or more washes to remove the detergent (in for example, PBS).

The decellularized tissue, now as used herein, the engineered tissue, may then be further processed or treated as needed: separated from the anchor(s); formed into a sheet or the like; cut into shape; stored in a suitable storage solution; lyophilized; and /or sterilized.

Some embodiments of the invention may include a degradable scaffold that can be seeded by extracellular matrix producing cells. The scaffold may be formed from fibrin, PLA, PGA, or other synthetic or biological polymer, and mixtures thereof. The ECM producing cells can be cultured with the scaffold, allowing the cells to produce ECM, which can in turn replace the degradable scaffold. Optionally, the scaffold can be manipulated or processed (as described in more detail below) to create alignment of the fibers in the ECM (e.g., an anisotropic matrix). The final product, preferably in the form of a sheet, may be decellularized using detergents, dehydrated (e.g., freeze drying), or fixed/crosslinked (e.g., glutaraldehyde fixation) to create a sheet of engineered tissue with or without cells. The engineered tissue may in turn be used to make a skirt, leaflets, a valve, or a coating or layer on medical devices intended for implantation. The engineered tissue of this embodiment may be used in any body lumen, including but not limited to an artery, a vein, or any body lumen that passes a body fluid (e.g., a ureter or a urethra).

### CELL SOURCE

Matrix-producing cells include, but are not limited to fibroblasts, embryonic stem cells, post-natal stem cells, adult stem cells, mesenchymal cells, interstitial cells, endothelial cells, smooth or skeletal muscle cells, myocytes (muscle stem cells), chrondocytes, adipocytes, fibromyoblasts, and ectodermal cells, including ductile and skill cells, hepatocytes, Islet cells, cells present in the intestine and other parenchymal cells, and osteoblasts and other cells forming bone or cartilage.

### CULTURING INGREDIENTS

One or more matrix-producing cells may be cultured in any nutrient, growth, or maintenance medium. Exemplary media or nutrients include but are not limited to one or more of the following: fibrinogen; fibrinogen-like material; thrombin; L-ascorbate acid or a phosphate derivative of L-ascorbate acid (e.g. Asc 2-P); serum; and growth factors, Dulbecco's Modified Eagle's Medium.^{®}. (DMEM), DMEM F12 Medium.^{®}., Eagle's Minimum Essential Medium.^{®}., F-12K Medium.^{®}., Iscove's Modified Dulbecco's Medium.^{®}., RPMI-1640 Medium.^{®}, insulin, and/or ascorbic acid.

### DECELLULARIZATION

The cultured cells are then decellularized using any process that results in a decellularized engineered tissue. As used herein decellularized refers to a construct that is decellularized such that it is substantially acellular, immune-resistant, and/or calcification resistant. Preferably, the construct is substantially acellular comprising less than 2% cells, less than 1% cells or contains no cells. The cells are intact cells. The cells can be living cells or dead cells. Exemplary processes include but are not limited to: WO 2007/025233; WO 2010/120539; Ott, et al (2008, Nat. Med., 14:213-21); Baptista, et al. (2009, Conf. Proc. IEEE Eng. Med. Biol. Soc., 2009:6526-9); or Crapo, et al., (2011, Biomaterials, 32:3233-43).

Some embodiments of the present invention may use the processes and engineered tissue as disclosed in the following:
2007/061800; WO 2007/092902; 2016/0203262; WO/2004/018008; WO 2004/101012; U.S. Patent 8,192,981; U.S. Patent 8,399,243; U.S. Patent 8,617,237; U.S. Patent 8,636,793; U.S. Patent 9,034,333; U.S. Patent 9,126,199; U.S. Serial No. 10/523,618; U.S. Serial No. 10/556,959; U.S. Serial No. 13/771,676; 2015/0012083; 2009/0319003; 2011/0020271; 2012/0230950; 2013/0013083; 2014/0330377; 2014/035805; 2017/0135805; 2017/0296323; 2017/0306292; USP 8198245; USP 9127242; USP 9556414; USP 9657265; and USP 9650603.

In the embodiments of the disclosure the engineered tissue may be characterized by one or more of the following: oriented fibers; thickness up to about 1 mm, preferably between about 100 µm and about 500 µm; non-immunogenic; no crosslinked polymers on the surface; a sheet or shape that is anisotropic; a sheet that is suitable for cutting into shapes, e.g., by scalpel, die, or laser; suppleness; suturability; no or little calcification upon implant; crosslinking density; collagen concentration; collagen density; remodeling proclivity; absorption; resorption; degradability, regions of greater stiffness; and regions of greater flexibility. In preferred embodiments of the invention, the engineered tissue has oriented fibers, suture retention, and tensile strength.

Some embodiments of the disclosure includes a medical device comprising a stent supporting an engineered tissue valve, wherein said medical device is configured into a "rook" shape. In some embodiments of the disclosure, the rook shape is adapted for placement in the right atrium. In some embodiments, the rook design is adapted for placement in the inferior vena cava or the superior vena cava.

Some embodiments of the disclosure may further include storing and/or sterilizing a medical device of the present invention. These embodiments may include preselected storage solution; preselected sterilization solution or technique; storage packaging; and/or sterilization packaging.

Preferred embodiments include a medical device of the packaged and ready to use by the surgeon or in the operating room.

The present disclosure also includes a method for treating venous valve failure or incompetency by delivering and installing a prosthesis of the present invention in an area of the anatomy suitable for providing therapeutic benefit.

Some embodiments of the disclosure include a therapeutically beneficial method by replacing a native valve. Some embodiments include a therapeutically beneficial method by treating the effects or symptoms of a venous valve failure or incompetency. In these embodiments of the disclosure, a medical device may be placed downstream of a failed or incompetent native valve. An exemplary embodiment includes but is not limited to the rook configuration, and treatment of a failed or incompetent tri-cuspid valve (whether native or previously replaced).

In some embodiments of the disclosure, the prosthesis is placed in a region of the common femoral vein. In preferred embodiments, the region(s) are places in the common femoral vein where an artery rests on or compresses the vein. Such compression may create localized areas of shape deflection or occlusion, which in turn, may cause valve incompetency and/or failure. Valve closest to heart is characterized by having the greatest head pressure. Inventors believe that repairing or replacing the top valve will/may improve function and competency of lower valves. Typically, the prostheses in these embodiments will comprise a stent with a valve or a stent with no valve.

Some embodiments also include a method and apparatus for repositioning and/or removing a prosthesis. In the embodiments of the disclosure that include one or more tethers, the tether may be captured or pulled in order to reposition the stent or prosthesis.

The embodiments of the present invention described below relate particularly to venous valve prostheses for treating valve failure, including but not limited to reduction of pressure effects. While the description sets forth various embodiment specific details, it will be appreciated that the description is illustrative only and should not be construed in any way as limiting the invention.

A prosthesis of the present disclosure is configured to be placed inside a channel of the body. In some embodiments, the prosthesis comprises a stent or latticework covered by or seeded with engineered tissue. In some embodiments, the prosthesis may further include a valve, with the prosthesis comprising one or more valves that allow fluid flow in one direction and prevents fluid flow in an opposite direction.

Each of the elements of the invention will now be described in more detail below.

A prosthesis comprises at least one tissue valve or at least one leaflet attached or secured to a support structure, wherein the support structure is collapsibly expandable. The tissue valve may include at least one leaflet attached to an intermediate region of the stent. The tissue valve is configured to permit fluid flow in a first direction and prevent fluid flow in an opposite direction. When the fluid flows in the first direction, the leaflet/valve is open having a flow-through opening.

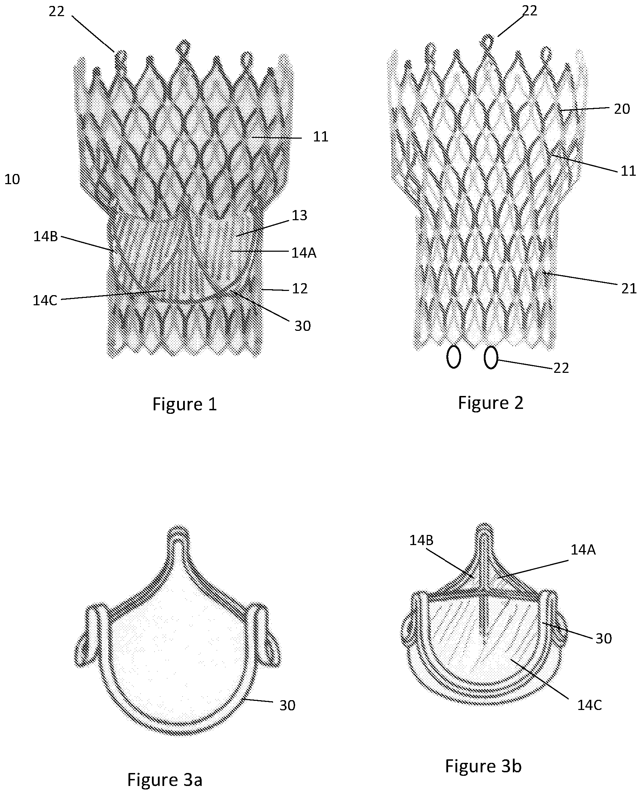

Figure 1 shows an exemplary embodiment of a prosthesis 10 of the present invention. In the illustrated embodiment, the prosthesis includes a framework or scaffold 11, a portion of which is covered by engineered tissue 12. This figure also shows a valve 13 comprising at least one leaflet 14 (or 14A, 14B, and 14C).

The tissue valve may include at least one leaflet 14 positioned within the lumen of the stent. The valve is configured to permit fluid flow in a first direction and prevent fluid flow in an opposite direction. When the fluid flows in the first direction, the leaflet 14 is open having a flow-through opening.

For illustrative purposes, valve leaflets are independent or separate flaps of material that are coupled to diametrically opposed locations within a tubular body and longitudinally extend within tubular body. However, in embodiments in accordance herewith the valve leaflets may be integrally formed together as a singular tubular component having a circular inflow opening and an elliptical outflow opening.

Tissue valves include at least one valve leaflet 14 (or 14A, 14B, and 14C). Sometimes, the tissue valve may have two, three or more leaflets. In preferred embodiments of the invention each valve includes three leaflets or cusps.

Stent 10 in general is constructed, formed, and positioned as is well known to one of ordinary skill in the art. In one embodiment, the stent 10 is self-expandable out of a delivery sheath. In operation, the stent is compressed radially to be held within the lumen of the delivery apparatus, sheath, catheter, applicator, or cannula. Upon delivery out of the apparatus, stents made from some materials (e.g., nitinol) self-expand to its pre-compressed state; with other stent materials (e.g., stainless steel) the stent may be manually expanded (e.g., by a balloon). The stent or scaffold may be formed on any material suitable for use in stent applications. Exemplary materials include but are not limited to stainless steel, nitinol, nitinol alloys, nickel titanium, plastic, polymer, a degradable material (e.g., fibrin, PLGA, PLA, PGA, cellulosic), or combinations thereof.

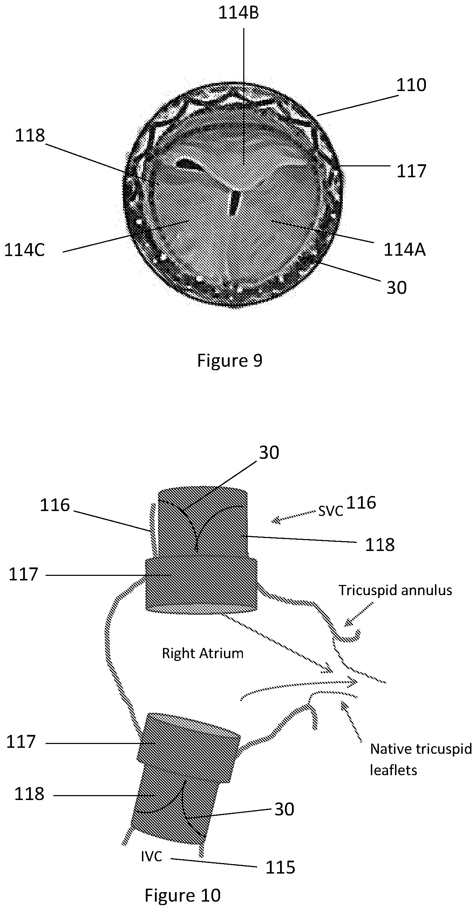

In preferred embodiments, the prosthesis or stent is pre-formed ex vivo into a predetermined shape. In most preferred embodiments of the invention, the predetermined shape conforms to or is suitable for placement in the desired location. For example, Figure 10 shows an embodiment of the invention configured for placement in the right atrium/vena cava interface or junction.

Pre-formed ex vivo into a predetermined shape is used herein to refer to conventional prosthesis stent construction that results a pre-determined shape when the prosthesis or stent is expanded. In accordance with the present disclosure, the prosthesis or stent may not be pre-formed; in these embodiments, the prosthesis or stent may expand or may be expanded to a desired shape or size (e.g., by self-expanding, or by balloon, etc.).

Because the shape is an approximate mirror image of the anatomy, the prosthesis or stent may be locked, fixed, restrained or the like in position without the need for barbs, hooks, needles, or the like.

While not intending to limit the invention to a particular size and shape, the inventors have found that it is desirable or advantageous to have a stent or prosthesis that is about 10% to about 20% larger than the target vessel where it is being placed. One skilled in the art will recognize that the size may vary or change because of a disease condition or the type of mammal being treated. an exemplary stent may be about 50 to about 110 mm in length, preferably from about 70 to about 90 mm; at its ends, about 12 to 20 mm in diameter, preferably about 14 to 17 mm; and at its widest, i.e., at a central portion of the stent adjacent the native tricuspid valve, about 40 to about 70 mm in width, preferably about 50 to about 60 mm.

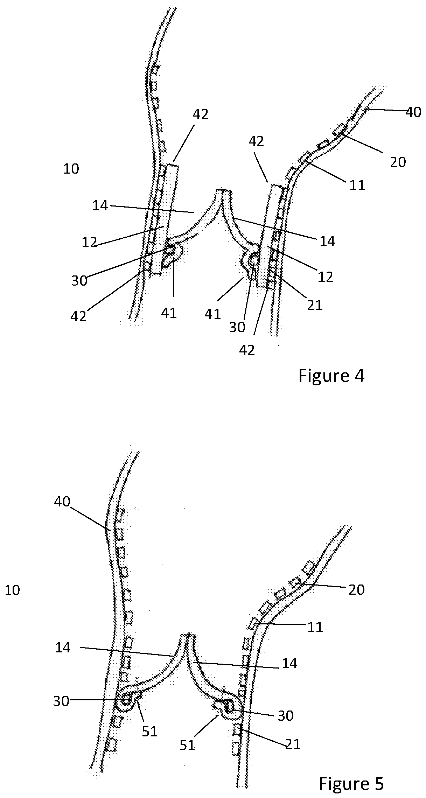

Figure 2 shows an exemplary stent 11. A stent in general is well known to comprise a latticework or assembly of struts that may be connected or unconnected, and that define a lumen or passage therethrough. In accordance with the present invention, a stent may include one or more sections that are configured and/or adapted to provide one or more desirable features of a prosthesis or stent. In the illustrated embodiment, section 20, the outflow section of the stent, is configured or shaped to conform to the target anatomy, such as a vein wall 40 (see Fig. 4). This feature is also shown in Figures 4, 5, and 10. Typically, the ability to conform to the anatomy may be provided by having a zone of greater flexibility, having latticework of greater flexibility, having, fewer connections between the struts, having a stent section formed of a different and more flexible material (than, for example zone 21), and various combinations thereof. In preferred embodiments, at least one section 20 is more flexible or contourable or moldable than another section of the stent in order to conform to the anatomy of the target vessel.

Section 20 of stent 11 may also be configured to anchor the stent/prosthesis in its intended position. Elements or structures for anchoring a stent or prosthesis are well known in the art. In the illustrated embodiment, section 20 is wider. In this and other embodiments, section 20 may also have greater expanding or radial force than other sections of the stent.

One or more sections of the stent, e.g., section 20 and/or section 21, may optionally include one or more repositioning elements 22. Elements 22 may be variously configured, as is shown by the different shapes illustrated in Fig. 2, 7, and 8. This element is adapted and configured to capture, recapture, position, reposition, and/or move the stent or prosthesis.

In the embodiments shown in Fig. 2, section 21 is configured and adapted to contain, house, or position other elements of the prosthesis, including but not limited to a skirt, one or more leaflets, and/or a valve. One skilled in the art will recognize that it may be desirable for this section to be stiffer or less flexible; in its construction and function, to support or facilitate the function and operation of the leaflets, skirt, and/or valve; exhibit higher radial strength or force; and may be designed or adapted to overstretch or the like to assure or improve apposition forces.

A prosthesis or stent may include a wireform adapted and configured to position and hold one or more leaflets in the stent, or to hold or position one or more valves in the stent. As is well known to those skilled in the art, the wireform's form and construction and function may be variously configured. An exemplary embodiment of a wireform is shown in Figures 3A and 3B. Figure 3A shows bare wireform 30. Figure 3B shows wireform 30 with leaflets 14 attached. Wireform 30 is configured to be attached, sewn, or connected to the interior of the stent and/or to one or more struts of the stent.

Figures 4 and 5 illustrate cross sections of a typical prosthesis of the present disclosure. In the illustrated embodiments, prosthesis 10 and/or stent 11 is configured to conform to or match the target anatomy 40. Prosthesis 10 comprises stent 11, one or more sections 20 and 21, wireform 30, and leaflets 14, all of which have been described above. Figure 4 shows a preferred embodiments in which the prosthesis 10 includes a skirt 12, even more preferably, a skirt formed from engineered tissue. Figure 5 shows an embodiment that includes leaflets or a valve and does not include a skirt. Figures 4 and 5 also show sutures 41 and 51 that may be used to connect or attach the leaflet to the wireform. Figure 4 also shows sutures 42 that may be used to connect or attach the skirt to the stent.

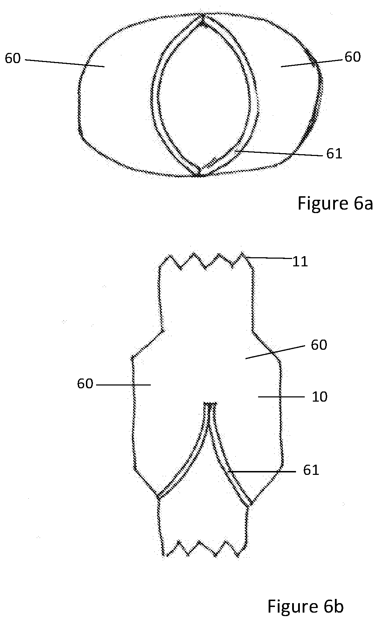

Figures 6A and 6B show an embodiment in which the prosthesis is configured to include one or more sinuses 60. Figure 6A shows a top view of prosthesis 10 that contains valve 61. As illustrated, the stent and valve configuration is designed to form one or more sinuses on the outflow side of the valve. In preferred embodiments the stent or prosthesis are oval or any other non-round shape that results in a sinus.

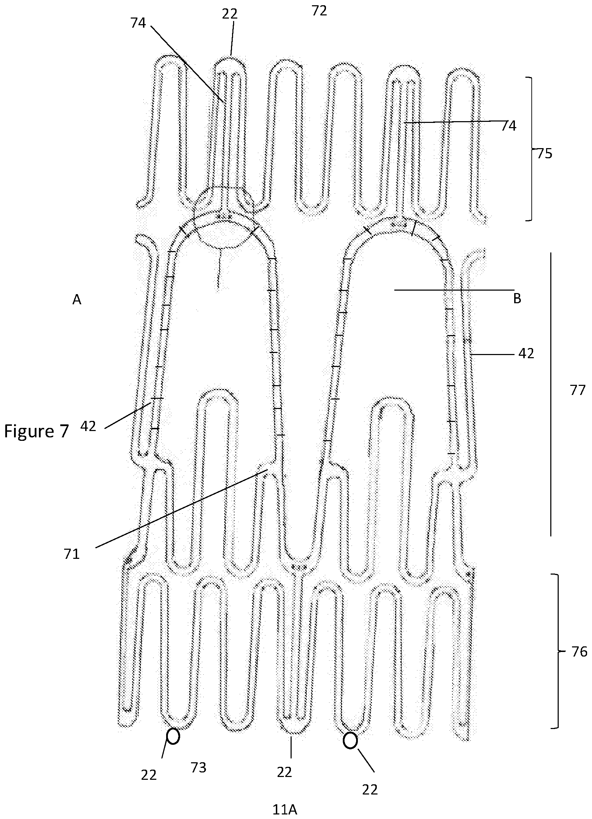

Figure 7 is an exemplary embodiment of a stent that is configured to produce a sinus. In the illustrated embodiment, stent 11A includes inflow side 72 and outflow side 73, and comprises a lattice work 71 configured to receive and position leaflets A and B (leaflet C not shown). As shown, the leaflets/valves are sewn to the struts using one or more sutures 42. The struts or stent may also include one or more connectors 74 that connect or attach to the base of the leaflet/valve. As shown, the leaflet is attached to the strut or stent with one point of attachment.

As noted above, a stent or prosthesis may include one or more zones having the same or different characteristics and/or functions. In the embodiment shown in Figure 7, the stent or prosthesis includes inflow ring 75, outflow ring 76, and a section between the inflow and the outflow that is configured to receive or position a valve, one or more leaflets, and/or a skirt (not shown). Each of these zones may include elements and function as described above for Figure 2.

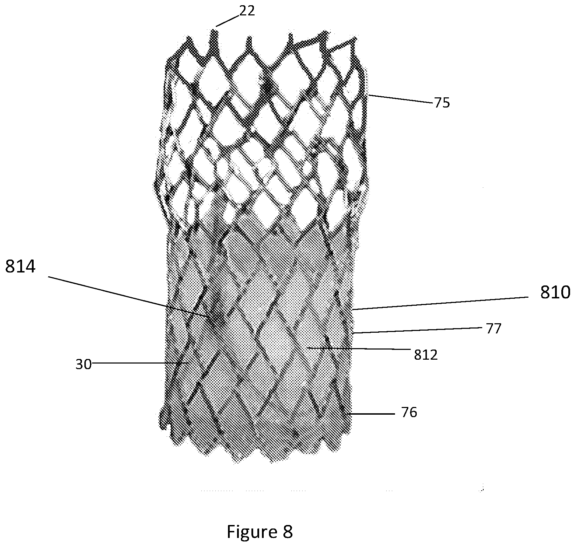

Figure 8 shows another embodiment of a stent/prosthesis of the present disclosure. Stent 810 positions a skirt 812 within an interior section or wall of the stent, along the circumference. The prosthesis further comprises wireform 30 to which may be attached one or more leaflets (not shown). The stent or prosthesis also may include one or more zones 75, 76, and 77 as previously described.

### ROOK EMBODIMENT

Figures 8-10 show an embodiment of a prosthesis configured and shaped to conform to the anatomy in the right atrium and the inferior vena cava (IVC) 115 or the right atrium and the superior vena cava (SVC) 116. This prosthesis also provides a check valve between the right ventricle and the IVC and/or the right atrium and the SVC. This venous valve also prevent blood from flowing from the right atrium/right ventricle into the venous system during ventricular systole. The prosthesis is shown in Figure 8; Figure 9 shows a view of the outflow side of the prosthesis of Figure 8; Figure 10 shows placement of two prostheses of Figure 8 in exemplary target anatomy, in this case, the superior vena cava/right atrium and the inferior vena cava/right atrium. In each prosthesis, blood flows into the prosthesis on the smaller diameter end, flows through the valve, then exits the prosthesis on the larger diameter end. The arrows show blood flow in the atrium into the native tricuspid valve.

This embodiment is a prosthesis adapted for treating tricuspid valve regurgitation or insufficiency, wherein the prosthesis comprises a self-expanding valve frame or stent, a skirt disposed within the valve frame; at least one leaflet attached to the skirt and/or stent; a portion of the stent on the outflow side having a larger diameter than the portion of the stent on the inflow side; and optionally, the stent or prosthesis may also include one or more elements for retrieving or repositioning the prosthesis. In preferred embodiments of the invention, the leaflet(s) may be supported by a wire frame, with said wireframe and leaflets being attached to the stent and/or skirt.

In the embodiment shown in Figures 8-10, stent 810 comprises an inflow side 118 and an outflow side 117, wherein the outflow side 117 is a larger diameter than the inflow side 118. In the illustrated embodiment, inflow end and outflow end are a unitary element. In other embodiments, stent 810 may comprise more than one segment or elements, e.g., an outflow segment and an inflow segment. These segments may be securely attached to each other, or may be loosely attached. In some embodiments, the outflow segment may be more flexible, moldable, or contourable, thereby allowing this segment to act as an anchor. Further, a more flexible portion allows this segment to conform to the shape and configuration of the anatomy.

In some embodiments of the stent, the inflow segment, or a portion thereof, may be more rigid or stiffer in the region where the leaflets/valve is attached, thereby supporting the valve and providing greater lumen diameter control. A stiffer region in inflow segment also aids in maintaining the intended shape of the valve/leaflets, thereby promoting overall function of the valve.

In more preferred embodiments, the prosthesis 910 will also comprise one or more of the following: valves that are available in different lengths, diameters to accommodate a patient's specific anatomy; valve frames designed or configured to be slightly larger than native vena cavea (i.e. designed with a parent diameter = VC diameter + 15%); valve frames designed with specific radial force to prevent damage to the VC or RA; valve frames with soft ends to prevent damage to venous lumen; contain a biomimetic material as the leaflet and skirt of the valve; the sealing ring would be covered with polyester, fixed tissue, or any other biomaterial could be used for any of the different parts of the invention being disclosed. For example: glutaraldehyde fixed pericardium (bovine, porcine, equestrian, etc.), synthetic materials (polyester, polyurethane, etc.); frames would all be ideally laser cut Nitinol, but could alternatively be braided Nitinol, or balloon expandable materials (i.e. laser cut or braided polished stainless steel).

Other optional design elements may include: opposing anchoring stent frame designed to have resulting forces opposing the right atrium walls and/or designed with a specific oversizing (i.e. 20% larger than native right atrium); valve frame would be designed with a specific oversized diameter (i.e. 10% larger than native tricuspid valve annulus); a sealing ring designed to oppose the wall of the annulus and parts of the atrium wall without occluding the coronary sinus; radial force of the self-expanding positioning frame would provide adequate force opposing the right atrium, thus anchoring the valve frame into place and to maintain position through all cycles of the heart. Radial force of the self-expanding valve frame would be sufficient to ensure adequate positioning in the annulus of the native tricuspid valve and prevent leakage; adhesion promotion: the stent may be designed with a specific surface finish (microporous, parylene coated, sand blasted, chemical etch) to promote adhesion of the stent to the wall of the right atrium; any embedded structures in the engineered tissue may have various surface finishes or coatings to promote adhesion of the engineered tissue; anchoring frame could be a coil to act as a spring against the right atrium wall; valve frame could be a braid; anchoring frame could be made of bio-absorbable or bioresorbable materials.

In the illustrated embodiment, the shaped prosthesis functions to treat or mediate tricuspid valve insufficiency without replacing the native tri-cuspid valve. This shaped prosthesis may also be used to treat other symptoms of tricuspid regurgitation, including but not limited to fatigue, abdominal swelling, abnormal heart rhythms, enlarged liver, and shortness of breath.

As used in relation to this embodiment, conforming to the anatomy refers to the shape of the prosthesis or valve frame, whereby a portion 117 of the prosthesis extends into the right atrium. In preferred embodiments, this portion 117 is larger is diameter than the portion 118 of the prosthesis that extends into IVC/SVC. Portion 117 with the larger stent diameter is on the outflow end of the prosthesis/valve and further provides a mechanism for anchoring the prosthesis in place during a systolic contraction.

### DELIVERY

The step of delivering either the prosthesis endoluminally is through any conventional method, including but not limited to an incision at a blood vessel selected from a group consisting of a jugular vein, a femoral vein, and a subclavian vein.

### Additional embodiments:

Rook designed valve frame used as anchoring mechanism along with self-expanding stent with parent diameter of up to 25% larger than the native landing zone in the target lumen.

Stent with non-atraumatic ends to prevent damage to the venous lumen (i.e. lower radial forces via thinner struts, additional blasting, longer radius on stent struts, encapsulated in tissue or fabric, etc.).

Stent with built in sinus to encourage wash out of areas prone to hemostasis.

Venous valve frame with inflow and outflow ring of struts with high radial force used for securing position, with a valve suspended between the two rows of struts (more specific to venous vein stents rather than vena cava).

Venous valve with soft inflow and outflow ends to prevent traumatic injury to venous lumen and a valve region having higher radial force capacity.

Venous valve frame with no stent structure behind leaflets outflow area, thus creating an opportunity for the native venous lumen to naturally create a sinus according to the flow dynamics during remodeling.

Venous valve frame embedded in engineered tissue to provide a biomimetic outer and inner surface thus preventing stenosis.

Venous valve frame with high flexibility and high radial force to accommodate external forces on the valve frame without compromising the integrity of the valve.

Venous valve frame made from bioresorbable material with attached engineered tissue leaflets.

A bioresorbable venous valve frame that is embedded within the engineered tissue to provide an inner and outer biomimetic surface which resorbs or absorbs into the surrounding tissue as the valve leaflets remodel.

A venous stent where the bottom of the leaflets make direct contact with the target vessel lumen wall thus creating a seal (as opposed to relying on a skirt to prevent paravalvular leakage). Essentially removing skirt in order to reduce profile of delivery.

Venous valve with inner wire form.

Venous valve without inner wire form.

Venous valve with bioresorbable/absorbable inner wire form within Nitinol stent valve frame.

Venous valve with bioresorbable/absorbable valve frame with non-resorbable inner wire form.

Venous valve frame with extended end to allow for a slow gradual deployment which also doubles as a way to partially (or fully) retrieve the device (funnel, "fingers").

A medical device that uses engineered tissue, thereby obviating the need to use anti-immune system agents (e.g., warfarin) or anti-thrombin agents when the medical device is implanted.

In all examples above, there will be a means of attaching a tether to allow for gradual deployment and/or retrievability.

A venous valve for implantation within a body lumen, comprising: an elongate valve frame configured to shift between a collapsed configuration and an expanded configuration, wherein the valve frame is configured to exert a radially outward force against a wall of the body lumen in the expanded configuration; wherein the valve frame includes an inner frame portion and an outer frame portion; a first valve leaflet and a second valve leaflet formed from engineered tissue and attached to the inner frame portion and defining a lumen through the valve, the first and second valve leaflets having an open configuration configured to permit flow of a bodily fluid therethrough and a closed configuration configured to restrict flow of the bodily fluid therethrough; wherein the inner frame portion defines a cross-sectional area of the valve relative to a central longitudinal axis of the lumen having a circular shape in the expanded configuration; wherein the outer frame portion defines a cross-sectional area of the valve relative to the central longitudinal axis of the lumen having an ovoid shape in the expanded configuration wherein the ovoid shape is defined by a first dimension and a second dimension each measured perpendicular to the central longitudinal axis of the lumen through the valve, wherein the first dimension is greater than the second dimension; wherein the outer frame portion defines a sinus pocket.

The medical device wherein the valve is configured to replace an existing valve structure within the body lumen.

The medical device wherein the valve is configured to augment an existing valve structure within the body lumen.

Engineered tissue comprising a degradable scaffold, wherein said scaffold is seeded with one or more matrix-producing cells and cultured in a medium that promotes the production of an extracellular matrix by the cells.

A medical device comprising a degradable scaffold or frame, wherein said degradable scaffold is replaced by extracellular matrix produced by the cells.

The prosthesis suitable for use in a body lumen comprising a degradable scaffold.

Embodiments of the present disclosure are directed to vascular medical devices, systems and methods for valve replacement and/or augmentation. Particularly, the present disclosure provides venous valve frames, venous valves that utilize the venous valve frames, and methods for forming and using the venous valve frame and the venous valve. Various embodiments of the present disclosure can be used to replace and/or augment an incompetent valve in a body lumen.

The valve frame can be formed in a wide variety of configurations. For example, the valve frame can include a first structural member and a second structural member that together form a unitary structure with an open frame configuration.

In an additional embodiment, the second structural member helps to define a bulbous portion of the valve frame. As illustrated, the second structural member extends radially and longitudinally from the outer surface of an area defined by the first structural member to form the bulbous portion.

The compressible nature of the valve can accommodate changes in body lumen size (e.g., diameter of the body lumen) by flexing to expand and/or contract to change the diameter of the valve frame. The frame can also provide sufficient contact and expansion force with the surface of a body lumen wall to encourage fixation of the valve and to prevent retrograde flow within the body lumen around the edges of the frame and the surface of a lumen when combined with a closed state of the valve leaflets attached thereto. Anchoring elements (e.g., barbs) can also be included with valve.

In one embodiment, the valve leaflets and the bulbous portion of the valve frame provide surfaces that define a sinus pocket. The sinus pocket provides a dilated channel or receptacle as compared to the elongate base portion of the venous valve. In one embodiment, the presence of the sinus pocket better ensures that the valve leaflets do not come into contact with a significant portion of the valve frame and/or the inner wall of the vessel in which the valve is implanted. For example, the sinus pocket can help prevent adhesion between the valve leaflets and the vessel wall due to the presence of a volume of blood there between.

The sinus pocket can also allow improved valve leaflets dynamics (e.g., opening and closing of the valve leaflets). For example, the sinus pocket can allow for pressure differentials across the surfaces of the valve leaflets that provide for more rapid closing of the valve leaflets as the retrograde blood flow begins.

The embodiments of the frame described herein can also be constructed of one or more of a number of materials and in a variety of configurations. The frame embodiments can have a unitary structure with an open frame configuration. The frame can also be self-expanding. Examples of self-expanding frames include those formed from temperature-sensitive memory alloy which changes shape at a designated temperature or temperature range, such as Nitinol. Alternatively, the self-expanding frames can include those having a spring-bias. In addition, the valve frame can have a configuration that allows the frame embodiments be radially expandable through the use of a balloon catheter. In this embodiment, the valve frame can be provided in separate pieces (e.g., two frame pieces) that are delivered individually to the implant site.

The device or stent can further include one or more radiopaque markers (e.g., rivets, tabs, sleeves, welds). For example, one or more portions of the frame can be formed from a radiopaque material. Radiopaque markers can be attached to, electroplated, dipped and/or coated onto one or more locations along the frame.

The material of the valve leaflets can be coupled to the valve leaflet connection location in a variety of ways so as to provide the various embodiments of the valve of the present disclosure. For example, a variety of fasteners can be used to couple the material of the valve leaflets to the frame so as to form the valve. Suitable fasteners can include, but are not limited to, biocompatible staples, glues, sutures or combinations thereof. In an additional embodiment, the material of the valve leaflets can be coupled to the frame through the use of heat sealing, solvent bonding, adhesive bonding, or welding the material of the valve leaflets to either a portion of the valve leaflets (i.e., itself) and/or the frame.

During the fusing process, mechanical stress may be used to induce cellular orientation and phenotypic modulation of the cells within the tissue. Thus, appropriate forces may be applied to maturating tissue in order to induce fiber orientation. Such forces may also prevent shrinkage and maintain the desired cell differentiation. Anchors may be used to maintain or create such desired cell differentiation and fiber orientation. The current invention provides a methods of anchoring maturing cultured tissues. In one embodiment, the method comprises an adjustable anchor means, preferably comprising a multiplicity of spaced apart anchors (such as moveable weights or ingots), wherein the anchors are suitable for (1) applying sufficient tension across the sheet of living tissue to prevent shrinkage and/or maintain cellular differentiation and/or induce orientation of cells in at least one sheet of living tissue and (2) allowing contraction of at least one sheet of living tissue once a predetermined threshold of tension is exceeded across the sheet of living tissue.

Combining cell traction forces with specific construct geometry can provide a specific collagen fiber orientation to better support valvular function of the later constructed valve, such as coaptation of the leaflets, reduced regurgitation, effective orifice area, and low pressure gradients, to name a few.

An exemplary tissue-engineered valve includes a plurality of tissue leaflets which are cut from tissue-engineered tissue. The leaflets are attached together to form a dimensionally stable and consistent coapting leaflet subassembly when subjected to physiological pressures. Then each of the leaflets of the subassembly is aligned with and individually sewn to a wireform, typically from the tip of one wireform commissure, uniformly around the leaflet cusp perimeter, to the tip of an adjacent wireform commissure. The wireform is usually covered with tissue-engineered tissue but can alternatively be covered with cloth or other material. The sewed sutures act like similarly aligned staples, all of which equally take toe loading force acting along the entire cusp of each of the pre-aligned leaflets. The resulting tissue-wireform structural assembly thereby formed reduces stress and potential fatigue at the leaflet suture interface by distributing stress evenly over the entire leaflet cusp from commissure to commissure.

Oval in shape, and tapered: e.g., 12 mm at the inflow end and 15 mm at the outflow. Preferred: round inflow side and oval outflow end.

Preferred: short side of stent approximately the same size as the ID of the vein; other side needs to be larger.

Stent design: softer/more flexible on both ends; middle region with stiffer. Such configuration mimics nature, e.g., ends with lower radial force.

The pressure differential that occurs during normal blood circulation between the blood on the valve inflow area and the gravity or back pressure on the valve outflow area allows the valve to operate as a one-way or flow check valve in a similar manner as a natural venous valve. The valve's resistance to opening, or cracking pressure, depends on various factors, including the valve leaflet material, the thickness of the valve leaflet material, thickening of the leaflets as the implant ages in vivo; and the geometry of the valve inflow area. By optimizing these factors, the valve is designed to open under inflow pressure conditions that depend on the particular implantation site of the prosthetic valve.

A stent is a cylindrical component that defines fluid passageway there through. In one embodiment, the tubular body is formed from a nitinol reinforced tissue or fabric, or preferably covered, coated, or seeded with engineered tissue. Frame is formed to be self-expanding to return to an expanded deployed configuration from a compressed or constricted delivery configuration, and in an embodiment may be formed from nitinol. Frame allows the valve prosthesis to be compressed and constrained in a radially collapsed state but, when unconstrained, the valve prosthesis will assume an expanded diameter. It will be understood by one of ordinary skill in the art that the valve tubular body may have any configuration that is suitable for forming a fluid passageway through a body lumen, including but not limited to a vein. As an alternative to being cylindrical, in another embodiment, the frame of the tubular body may have an elliptical or oval cross-section at the midsection thereof. The distal and proximal ends of the frame and the tubular body are typically circular. The valve, when open, creates an elliptical channel with a sinus behind each leaflet. This narrowed channel effect will act to accelerate blood flow through the valve, which may help prevent thrombosis.

In embodiments hereof, the mating free edges of the valve leaflets may be pre-formed in order to ensure that they bend and seal in a consistent manner when the valve is in a closed configuration. At least the target edges of the valve leaflets may be placed into a die or mold and heat is applied in order to impart a shape memory thereto.

In another embodiment, in addition to or as an alternative to the shape imparted onto the free edges, a reinforcing element may be coupled to the edges of the leaflets in order to ensure they bend and seal in a consistent manner. For example, a valve has a tubular body and a pair of valve leaflets having free edges that meet in a commissure. A reinforcing wire is embedded within free edges of leaflets. The wire can be sewn or stitched in place or sandwiched between two layers of material.

A prosthesis or stent may also include one or more structures adapted or configured to position, reposition, or retrieve the prosthesis or stent. These structures include, but are not limited to tether(s); hooks, eyes, loop(s); a wide variety of male female elements suitable to connect with a corresponding or complementary female/male element on a catheter system.

The tether may be any thread, wire, filament, cord, fiber, strand, or any equivalent structures that is suited to the purpose of pulling on the prosthesis or stent. The typical tether may be a polymer, preferably a high density polyolefin. The tether may be any length.

Embodiments of the valve prostheses described herein are preferably delivered in a percutaneous, minimally invasive manner and may be delivered by any suitable delivery system. In contrast to surgically placed valves that require incisions and suturing at the site of a native valve, percutaneous transluminal delivery of a replacement valve can mitigate thromboses formed from an injury response. In general, a venous valve prosthesis having a self-expanding tubular body is loaded into a sheathed delivery system, compressing the self-expanding tubular body. The delivery system includes a retractable sheath having a proximal end and a distal end, and an inner shaft having a proximal end and a distal end terminating in a distal tip. Distal tip may be tapered and flexible to provide trackability in narrow and tortuous vessels. Sheath defines a lumen extending there through, and inner shaft slidably extends through the lumen of sheath. In an embodiment, inner shaft may define a guidewire lumen for receiving a guidewire therethrough or may instead be a solid rod without a lumen extending therethrough.

Sheath covers and radially constrains the valve prosthesis while the delivery system is tracked through a body lumen to the deployment site. Sheath is movable in an axial direction along inner shaft and extends to a proximal portion of the delivery system where it may be controlled via an actuator to selectively expand the valve prosthesis. When the actuator is operated, sheath is retracted over inner shaft in a proximal direction. When sheath is proximally retracted with respect to the hub of the delivery system, the self-expanding valve prosthesis is released and allowed to assume its expanded configuration. An exemplary suitable delivery system is described in U.S. Pat. No. 7,264,632 to Wright et al.

The present disclosure includes a method of controlling flow through a vein by percutaneously delivering a prosthetic venous valve of the present invention to a treatment site within the vein. As described in the above embodiments, prosthetic venous valve includes a self-expanding tubular body defining a fluid passageway and a pair of opposing leaflets coupled to an interior surface of the tubular body. The leaflets of the prosthetic venous valve are biased in a closed configuration in which free edges of the leaflets seal along a sinusoidal or zigzagged commissure to prevent retrograde blood flow through the fluid passageway of the tubular body. Optionally, the prosthetic venous valve may include one or more radiopaque or echogenic markers thereon in order to aid in positioning the valve prosthesis to span across an incompetent native valve.

A delivery system is percutaneously introduced into the patient's vasculature. Delivery system is then threaded or tracked through the vascular system of the patient until the prosthetic venous valve is located within a predetermined target site, such as proximate to or over an incompetent native valve within a vein. More particularly, the prosthetic venous valve may be utilized to replace a native incompetent valve or may be deployed between two native valves. When utilized to replace a native incompetent valve, the tubular body of the valve is configured to press radially against the native valve leaflets to hold the native valve leaflets against walls of the native valve annulus and/or against walls of an adjacent lumen to thereby prevent the native valve leaflets from obstructing blood flow through the prosthetic valve. Thus, the prosthetic venous valve may be implanted without requiring removal of a native valve from the vein. In addition, since the tubular body of the prosthetic venous valve spans across the insufficient native valve, the prosthetic venous valve is expected to arrest the progressive damage to the vein caused by the marginal function of the native valve. Blood flow will be directed through the fluid passageway of the prosthetic venous valve and thus bypass any distended or bulged area of the native valve. The damaged venous wall will thus be protected and allowed to scar and/or heal.

Although the valve prosthesis is described herein as self-expanding for percutaneous placement, it should be understood that the prosthetic venous valve may alternatively be surgically implanted within a vein in a non-percutaneous manner and may be anchored to the vein in any suitable manner, such as via sutures, clips, or other attachment mechanisms. For example, in such a surgical embodiment, the tubular body of the prosthetic venous valve may include a series of apertures through which sutures can be passed.

Embodiments of the valve prostheses described herein may include an anti-coagulant coating on one or more blood-contacting surfaces of the tubular body and/or the valve leaflets in order to mitigate the formation of thrombus on foreign materials in the bloodstream. In one embodiment, an anti-coagulant material may be embedded in the material of the tubular body and/or the valve leaflets. The anti-coagulant material may be heparin, coumadin, aspirin, ticlopidine, clopidogrel, prasugrel or other suitable anti-coagulant pharmaceuticals.

While various embodiments according to the present invention have been described above, it should be understood that they have been presented by way of illustration and example only, and not limitation. It will be apparent to persons skilled in the relevant art that various changes in form and detail can be made therein without departing from the scope of the invention. Thus, the breadth and scope of the present invention should not be limited by any of the above-described exemplary embodiments, but should be defined only in accordance with the appended claims.

### Definitions

The following definitions are used in reference to the invention:
"Distal" or "distally" are a position distant from or in a direction away from the clinician. "Proximal" and "proximally" are a position near or in a direction toward the clinician. In addition, the term "self-expanding" is used in the following description with reference to a tubular body or frame of the valves hereof and is intended to convey that the structures can be provided with a mechanical memory to return the structure from a compressed or constricted delivery configuration to an expanded deployed configuration.

Non-exhaustive exemplary self-expanding materials suitable for such structures include stainless steel, a pseudo-elastic metal such as a nickel titanium alloy (nitinol), various polymers, or a nickel-cobalt-chromium-molybdenum superalloy, or other metal. Mechanical memory may be imparted to a wire or tubular structure by thermal treatment to achieve a spring temper in stainless steel, for example, or to set a shape memory in a susceptible metal alloy, such as nitinol. Various polymers that can be made to have shape memory characteristics may also be suitable for use in embodiments hereof, including polymers such as polynorborene, trans-polyisoprene, styrene-butadiene, cross-linked polycyclooctine and polyurethane.

Although the description of the invention is in the context of treatment of blood vessels such as veins, the invention may also be used in any other body passageways where it is deemed useful.

In order to mimic the structure of a native venous valve, leaflets #, # form a substantially elliptical or oval outflow opening # when the valve leaflets are in the open configuration (shown in FIG. #). As used herein, "substantially elliptical" is intended to mean that outflow opening # is not circular but rather in a generally oval or elliptical shape such that the length of a major axis of the opening is greater than the length of a minor axis of the opening. For description purposes, outflow opening # is described herein as elliptical but the outflow opening is not required to be an exact geometric ellipse. Elliptical outflow opening mimics the structure of a native venous valve because it creates sinus pockets or sinuses behind each open leaflet #, #, respectively. Sinuses #, # are formed between the outer surface of valve leaflets and the inner surface of tubular body #. In addition to providing sinuses that mimic a native venous valve, elliptical outflow opening also mimics the luminal narrowing found in native valves.

Venous valve failure as used herein refers to any condition or cause that allows blood to flow in a direction contrary to the function of a healthy natural venous valve. A typical valve failure includes but is not limited to regurgitation, an event well known to those skilled in the art.

While the invention has been described in some detail by way of illustration and example, it should be understood that the invention is susceptible to various modifications and alternative forms, and is not restricted to the specific embodiments set forth in the Examples.

## Claims

1. An engineered tissue venous valve comprising a decellularized engineered tissue tube defining a fluid flow path through the tube, **characterized in that** the engineered tissue is formed from a suspension or gel comprising fibrinogen, thrombin, and extracellular matrix (ECM)-producing cells, which contracts in a controlled fashion to orient fibers in a certain direction such that the engineered tissue has aligned fibers.

2. The engineered tissue venous valve of claim 1 wherein the engineered tissue further comprises one or more leaflets.

3. The engineered tissue venous valve of claim 1 wherein the engineered tissue further comprises a skirt.

4. The engineered tissue venous valve of claim 1 further comprising a stent, and the venous valve further comprises a valve disposed within the stent and formed from engineered tissue.

5. The engineered tissue venous valve of claim 4 further comprising a skirt formed from engineered tissue and disposed on a radial portion of the stent adjacent the valve.

6. The engineered tissue venous valve of claim 1 wherein the tube further comprises a first zone that is on the inflow side of the tube; a second zone is on the outflow side of the frame; and a third zone between the first and second zone, said third zone being configured to position a valve.

7. The engineered tissue venous valve of claim 6 wherein one or both of said first zone and second zone are shaped to conform to the anatomical shape of a vein.

8. The engineered tissue venous valve of claim 1 wherein said venous valve is shaped to conform to the anatomy in a junction between a vena cava and the right atrium; wherein said tube comprises a skirt formed from engineered tissue and disposed on a radial portion of the tube; an engineered tissue valve disposed within the tube and the skirt; and wherein said tube comprises a first region on the outflow end and shaped to conform to the target anatomy; and a second region adapted to position the skirt and valve.

9. The engineered tissue venous valve of claim 1 wherein the frame is degradable.

## Patentansprüche

1. Eine Venenklappe aus künstlichem Gewebe, die einen Schlauch aus dezellularisiertem künstlichem Gewebe aufweist, einen Flüssigkeitsströmungsweg durch den Schlauch definierend,
**dadurch gekennzeichnet, dass**
das künstliche Gewebe aus einer Suspension oder einem Gel gebildet wird, das Fibrinogen, Thrombin und eine extrazelluläre Matrix (EZM = extracellular matrix) produzierende Zellen aufweist, die sich auf eine kontrollierte Weise zusammenzieht, um Fasern in einer bestimmten Richtung auszurichten, so dass das künstliche Gewebe ausgerichtete Fasern aufweist.

2. Die Venenklappe aus künstlichem Gewebe nach Anspruch 1, wobei das künstliche Gewebe ferner ein oder mehrere Segel aufweist.

3. Die Venenklappe aus künstlichem Gewebe nach Anspruch 1, wobei das künstliche Gewebe ferner einen Schirm aufweist.

4. Die Venenklappe aus künstlichem Gewebe nach Anspruch 1, die ferner einen Stent aufweist, und wobei die Venenklappe ferner eine Klappe aufweist, die innerhalb des Stents angeordnet ist und die aus künstlichem Gewebe gebildet ist.

5. Die Venenklappe aus künstlichem Gewebe nach Anspruch 4, die ferner einen Schirm aufweist, der aus künstlichem Gewebe gebildet und auf einem radialen Abschnitt des Stents benachbart zur Klappe angeordnet ist.

6. Die Venenklappe aus künstlichem Gewebe nach Anspruch 1, wobei der Schlauch ferner Folgendes aufweist:
eine erste Zone, die sich auf der Einströmseite des Schlauchs befindet; eine zweite Zone, die sich auf der Ausströmseite des Rahmens befindet; und eine dritte Zone zwischen der ersten und der zweiten Zone, wobei die dritte Zone so konfiguriert ist, dass sie eine Klappe positioniert.

7. Die Venenklappe aus künstlichem Gewebe nach Anspruch 6, wobei eine oder beide der ersten und der zweiten Zone so geformt sind, dass sie der anatomischen Form einer Vene entsprechen.

8. Die Venenklappe aus künstlichem Gewebe nach Anspruch 1, wobei die Venenklappe so geformt ist, dass sie der Anatomie in einer Verbindung zwischen einer Hohlvene und dem rechten Vorhof entspricht;
wobei der Schlauch einen aus künstlichem Gewebe geformten Schirm aufweist, der an einem radialen Abschnitt des Schlauchs angeordnet ist; wobei eine Klappe aus künstlichem Gewebe innerhalb des Schlauchs und des Schirms angeordnet ist; und
wobei der Schlauch einen ersten Bereich am Ausflussende aufweist und wobei dieser so geformt ist, dass er der Zielanatomie entspricht; und einen zweiten Bereich, der so angepasst ist, dass er den Schirm und die Klappe positioniert.

9. Die Venenklappe aus künstlichem Gewebe nach Anspruch 1, wobei der Rahmen abbaubar ist.

## Revendications

1. Valve veineuse obtenue par génie tissulaire comprenant un tube en tissu obtenu par génie tissulaire décellularisé définissant une voie d'écoulement de fluide à travers le tube, **caractérisé en ce que** le tissu obtenu par génie tissulaire est formé à partir d'une suspension ou d'un gel comprenant du fibrinogène, de la thrombine et des cellules productrices de matrices extracellulaires (MEC), qui se contracte de manière contrôlée pour orienter les fibres dans une certaine direction, de sorte que le tissu obtenu par génie tissulaire présente des fibres alignées.

2. Valve veineuse obtenue par génie tissulaire selon la revendication 1, dans laquelle le tissu obtenu par génie tissulaire comprend en outre un ou plusieurs feuillets.

3. Valve veineuse obtenue par génie tissulaire selon la revendication 1, dans laquelle le tissu obtenu par génie tissulaire comprend en outre une jupe.

4. Valve veineuse obtenue par génie tissulaire selon la revendication 1, comprenant en outre une endoprothèse, et la valve veineuse comprend en outre une valve disposée à l'intérieur de l'endoprothèse et formée à partir de tissu obtenu par génie tissulaire.

5. Valve veineuse obtenue par génie tissulaire selon la revendication 4, comprenant en outre une jupe formée à partir de tissu obtenu par génie tissulaire et disposée sur une partie radiale de l'endoprothèse adjacente à la valve.

6. Valve veineuse obtenue par génie tissulaire selon la revendication 1, dans laquelle le tube comprend en outre une première zone qui se trouve du côté de l'entrée du tube ; une deuxième zone se trouve du côté de la sortie du cadre et une troisième zone entre la première et la deuxième zone, ladite troisième zone étant configurée pour positionner une valve.

7. Valve veineuse obtenue par génie tissulaire selon la revendication 6, dans laquelle ladite première zone et ladite seconde zone, ou l'une des deux, sont formées pour épouser la forme anatomique d'une veine.

8. Valve veineuse obtenue par génie tissulaire selon la revendication 1, où ladite valve veineuse est formée pour se conformer à l'anatomie d'une jonction entre la veine cave et l'oreillette droite ; où ladite tube comprend une jupe formée à partir de tissu obtenu par génie tissulaire et disposée sur une partie radiale du tube ; une valve obtenue par génie tissulaire disposée à l'intérieur du tube et de la jupe ; et où ladite tube comprend une première région sur l'extrémité d'écoulement et façonnée pour se conformer à l'anatomie cible ; et une deuxième région adaptée pour positionner la jupe et la valve.

9. Valve veineuse obtenue par génie tissulaire selon la revendication 1, où le cadre est dégradable.
